# FASCICULE DE BREVET EUROPEEN

(11) **EP 1 874 262 B1**
(45) Date de publication et mention de la délivrance du brevet: **08.12.2010**
(21) Numéro de dépôt: 06755446.9
(22) Date de dépôt: 21.04.2006
(51) Int. Cl.: A61K 8/04, A61K 8/81, A61Q 3/02

(54) **COMPOSITION AQUEUSE POUR VERNIS A ONGLES**
WÄSSRIGE NAGELLACKZUSAMMENSETZUNG
AQUEOUS NAIL VARNISH COMPOSITION

(30) Priorité: 29.04.2005 FR 0504376
(43) Date de publication de la demande: 09.01.2008
(73) Titulaire: Fiabila, 28130 Maintenon (FR)
(72) Inventeur: MALNOU, Alain, F-27350 Routot (FR)
(74) Mandataire: Le Cloirec, Claudine
(86) Numéro de dépôt international: PCT/FR2006/000893
(87) Numéro de publication internationale: WO 2006/117449

(56) Documents cités:
- EP-A- 0 752 244
- EP-A- 1 371 685
- EP-A- 1 371 693
- FR-A- 2 733 147
- US-A- 5 922 334
- DATABASE EPODOC EUROPEAN PATENT OFFICE, THE HAGUE, NL; 6 avril 1992 (1992-04-06), SUGAWARA SUSUMI ET AL.: "Water -Based Manicure Cosmetic" XP002362441 & JP 04 103513 A (KAO CORP) 6 avril 1992 (1992-04-06)
- DATABASE WPI Section Ch, Week 199502 Derwent Publications Ltd., London, GB; Class A13, AN 1995-011726 XP002362443 & JP 06 298624 A (MITSUI TOATSU CHEM INC) 25 octobre 1994 (1994-10-25)

## Description

La présente invention concerne une composition pour vernis à ongles, notamment une composition aqueuse.

Les vernis à ongles traditionnels, à base de résines nitrocellulosiques, contiennent de fortes proportions de solvants organiques. De ce fait, ces vernis sont inflammables avec quelques risques de toxicité.

Comme pour les peintures, les formulateurs ont cherché à mettre au point des vernis sur une base aqueuse. Pour cela, il faut résoudre les trois principaux problèmes que sont la vitesse de séchage et de durcissement du film, la brillance du film obtenu et l'adhérence de ce film sur l'ongle.

Les solutions les plus fréquemment proposées sont basées sur l'utilisation de dispersions ou suspensions de polymères dans l'eau, de préférence des suspensions de copolymères acryliques, vinyliques, polyesters, polyuréthanes, etc.

Pour former un film cohérent, les particules de ces suspensions doivent coalescer, avec ou sans l'aide de coalescents et/ou de plastifiants. Les solvants coalescents s'évaporent lors du séchage, les solvants plastifiants restent plutôt dans le film.

Pour résoudre ces problèmes, les auteurs ont testé l'utilisation de suspensions de divers polymères, de différentes tailles et leurs mélanges, de suspensions de différentes duretés (températures de transition vitreuse) coalescées et/ou plastifiées par des solvants de différentes natures, ayant différentes températures d'ébullition, de suspensions de différentes compositions (acryliques, vinyliques, polyuréthanes) ou utilisant des monomères particuliers voir par exemple les documents FR-A-2733147, US 5922334.

Le demandeur a découvert qu'une composition aqueuse présentant des propriétés acceptables pour une utilisation en vernis à ongles pouvait être obtenue à partir d'un mélange de polymères avec des caractéristiques particulières.

La composition aqueuse pour vernis à ongles selon la présente invention est donc **caractérisée en ce qu**'elle comprend un mélange
d'au moins une suspension aqueuse (A) de nanoparticules dures, de taille de 40 à 80 nm, d'un polymère acrylique à température de transition vitreuse supérieure ou égale à 55° C, en combinaison avec un plastifiant dudit polymère choisi parmi l'adipate de dibutyle, le sébacate de dibutyle, l'acétyl citrate de tributyle, le citrate de triéthyle, ou un butyrate d'alkyldiol, ou un mélange de ceux-ci ;
et d'au moins une suspension aqueuse (B) de nanoparticules, de taille de 50 à 80 nm de polymère acrylique préréticulé, renfermant des monomères di- ou trifonctionnels et présentant une température de formation de film inférieure à 15° C.

La composition aqueuse pour vernis à ongles selon l'invention peut également renfermer une solution (C) d'un polymère acrylique de faible poids moléculaire inférieur à 20 000 environ, à fort indice d'acide, neutralisée à un pH entre 7 et 8,5 environ.

Les nanoparticules de la suspension (A) sont de préférence à base d'un polymère de poids moléculaire supérieur à 200 000 environ, et présentent une taille de 40 à 80 nanomètres environ.

La suspension aqueuse (A) est préalablement plastifiée, en particulier un plastifiant du polymère de cette suspension. En effet, pour pouvoir former un film, cette suspension (A) doit être "ramollie" avec un ou plusieurs solvants prédéfinis pour abaisser la température de formation du film. Ces solvants ne déstabilisent pas la suspension et sont stables dans l'eau. Après la formation du film, le solvant coalescent du polymère de la suspension va s'évaporer lors du séchage et redonner au film la dureté initiale de la suspension. A la différence du solvant dit coalescent, le plastifiant reste dans le film et participe au compromis dureté/souplesse du film final.

De manière avantageuse, la suspension aqueuse (A) est combinée à un solvant coalescent du polymère de la suspension (A). Le coalescent est un éther de glycol choisi parmi le propylène glycol n-butyl éther, le dipropylène glycol méthyl éther, le dipropylène glycol diméthyl éther, le tri propylène glycol méthyl ether ou un mélange de ceux-ci et le plastifiant est choisi parmi un butyrate d'alkyldiol (tel que le monoisobutyrate de triméthyl-pentanediol), l'adipate de dibutyle, le sébacate de dibutyle, l'acétyl citrate de tributyle, le citrate de triéthyle, ou un mélange de ceux-ci.

Les solvants coalescents et/ou plastifiants sont utilisés chacun dans des proportions jusqu'à 15 %, de préférence comprises entre 5 et 15 % en poids par rapport au polymère de la suspension (A).

La suspension (A) utilisée pour la présente invention présente de préférence un indice d'acide faible (mais avantageusement supérieur à environ 60 milligrammes de KOH par gramme de polymère) et est neutralisée par de l'ammoniaque.

De manière avantageuse, les nanoparticules de la suspension (B) présentent un taux de réticulation compris entre 0,5 et 5 %, et une taille de 50 à 80 nanomètres environ. Par taux de réticulation, on entend le taux de monomères diacrylates, par exemple, présents dans cette suspension. Une telle suspension donne des résultats intéressants en terme de rapidité de séchage car la présence de monomères di ou trifonctionnels permet de créer un réseau tridimensionnel rapidement, en favorisant la polymérisation.

Les monomères et polymères acryliques des suspensions et solutions décrites ci-dessus sont de préférence à base de styrène, de méthyl styrène, d'acrylate de méthyle, de méthacrylate de méthyle, d'acrylate de butyle, de méthacrylate de butyle, de méthacrylate d'hydroxyéthyle, de méthacrylate d'hydroxypropyle, ou un mélange de ceux-ci.

De préférence, la suspension (A) est riche en acrylate de méthyle ou en méthacrylate de méthyle. Les suspensions renferment au moins 40 % en solide de monomères ou de polymères.

La solution (C) du mélange pour composition aqueuse de la présente invention renferme un polymère acrylique de faible poids moléculaire ; par faible poids moléculaire on entend une masse molaire inférieure à 20 000 environ. La masse molaire préférée est comprise entre 9 000 et 18 000. Cette solution aqueuse présente un fort indice d'acide, c'est-à-dire supérieur à 200 milligrammes KOH par gramme de polymère, de préférence de 200 à 250 milligrammes KOH par gramme. Cette solution est neutralisée à l'aide d'une base forte jusqu'à un pH compris entre 7 et 8,5. La base forte est avantageusement volatile et peut être par exemple de l'ammoniaque. Une fois neutralisé, le polymère acrylique riche en acide devient soluble dans l'eau, tandis qu'au séchage l'ammoniaque s'évapore et le film redevient résistant à l'eau. Les deux suspensions acryliques sont obtenues par polymérisation directe dans l'eau, sans tensioactif, de mélange de différents monomères précités.

La composition aqueuse pour vernis à ongles selon l'invention renferme avantageusement entre 12 et 24 % en poids en poids de nanoparticules de la suspension (A), entre 8 et 16 % en poids de nanoparticules de la suspension (B), et entre 0,2 et 1 % en poids de polymère sec de l'éventuelle solution (C).

Si la quantité de nanoparticules de (A) est inférieure à 12 % en poids, le vernis séché se ramollit avec le temps ; si elle est supérieure à 24 %, le durcissement est long et le démaquillage difficile.

Pour une quantité de nanoparticules de (B) inférieure à 8 % en poids, le vernis est long à sécher et pour une quantité supérieure à 16 % en poids, le film séché est moins cohérent et a tendance à se ramollir avec le temps.

La solution (C) peut être omise quand on prépare un vernis incolore, bien qu'elle améliore l'adhérence et la formation du film par son action coalescente. Lorsqu'on l'utilise, il est préférable de ne pas dépasser 1 % en poids exprimé en polymère sec : s'agissant d'une solution aqueuse, elle est plus longue à sécher et est sensible à l'eau. Elle est très utile quand on prépare des vernis colorés car elle a de bonnes propriétés de dispersion des pigments.

Outre les suspensions (A) et (B) et la solution (C), la composition aqueuse selon l'invention peut renfermer également des colorants solubles dans l'eau et/ou des pigments dispersés dans la solution aqueuse acrylique (C).

La composition peut également renfermer des agents épaississants minéraux ou organiques, des agents antimousses siliconés ou non, des agents conservateurs, des agents d'étalement et/ou de glissance, des parfums, des neutralisants, ou des additifs actifs pour les soins des ongles.

Les dispersions réalisées avec les pigments classiques autorisés en cosmétique sont des dispersions stables sans sédimentation ni déphasage.

Les vernis à ongles fabriqués à partir de ces compositions présentent un temps de séchage du même ordre qu'un vernis avec des solvants, des caractéristiques de dureté en trois heures, un très bon brillant et une tenue sur l'ongle correcte pendant trois jours. Cette bonne tenue est la résultante d'un bon compromis dureté/souplesse, d'une bonne adhérence et d'une bonne résistance du film à l'eau. Le démaquillage reste facile avec un dissolvant classique à base de cétones et d'acétates.

Les exemples suivants permettent d'illustrer, de manière non limitative, la présente invention. Les constituants des différentes compositions de vernis testées sont regroupés dans le tableau 1 (exprimés en poids de la composition finale).

Dans les exemples ci-dessous la suspension (A) est d'abord plastifiée avec le coalescent et/ou le plastifiant avant d'être mélangée avec les autres constituants de la formule du vernis à ongles. De préférence les pigments sont dispersés dans la solution aqueuse acrylique (C) au moyen d'un broyeur à micro-billes.

L'eau déminéralisée est utilisée pour les suspensions (A) et (B), la solution (C), les différentes dispersions d'épaississant et de pigments et pour la mise à viscosité finale des compositions.

Les constituants Joncryl sont commercialisés par la Société Johnson Polymer B.V. :
- Le Joncryl 538 est une suspension aqueuse de polymères acides et esters (métt)acryliques/styrène présentant une température de formation de film (TMF) de 60° C et une température de transition vitreuse (Tg) de 64° C.
- Le Joncryl 8224 est une suspension acrylique présentant une TMF de 10° C et une Tg voisine de 46° C.
- Le Joncryl HPD71 E est une solution aqueuse d'un sel d'ammonium de polymères styrène/acides et esters (métt)acryliques.

Les exemples 1, 3 et 4 sont des vernis à ongles colorés selon l'invention, destinés à être appliqués directement sur l'ongle en deux couches successives.

L'exemple 2 est un vernis de soins (renfermant de la vitamine E) des ongles, destiné à être utilisé comme couche de base sous un vernis classique contenant des solvants.

Les suspensions (A) des compositions des exemples 1, 2 et 3 sont mélangées avec un coalescent et un plastifiant.

La suspension (A) de la composition de l'exemple 4 est mélangée sans coalescent, c'est-à-dire que le vernis final est sans composé organique volatil.

Les exemples 5 et 6 sont des exemples comparatifs.

L'exemple 5 est une composition de l'art antérieur ne renfermant pas de suspension pré-réticulée, mais une suspension conventionnelle non réticulée Joncryl SCX 1537 de même Tg (Température de transition vitreuse) (ici Tg = 46° C) en vue de présenter une dureté de film équivalente.

L'exemple 6 est un exemple de vernis sans suspension (A), mais avec uniquement une suspension réticulée (B) en présence d'une solution de type (C) pour la dispersion des pigments, et d'un peu de coalescent pour solubiliser les additifs.

Différents tests ont été réalisés à partir de ces compositions :
Les viscosités sont mesurées au viscosimètre Brookfield avec l'aiguille N°3 à 25° C à 6 tours/minute et à 60 tours/minute.

La stabilité est jugée après un mois à 50° C.

Les applications sont faites avec un applicateur de 100 micromètres liquide sur verre pour la mesure de la dureté et sur une carte de type "Leneta" pour la mesure du temps de séchage.

Le séchage est mesuré par la trace laissée par une bille glissant sur le vernis appliqué sur une carte "Leneta" laissée sur plaque chauffante à 35° C.

La dureté est mesurée à l'aide d'un pendule de "Persoz" sur un film appliqué sur une plaque de verre et séché 3 heures et 24 heures à température ambiante (20° C).

L'adhérence est jugée après "griffage" du film appliqué sur la plaque de verre selon un quadrillage de 1 mm.

Le vernis est ensuite testé en tenue sur les ongles pour juger l'usure prématurée, la rétention de brillant, l'écaillage et le décollement dans l'eau.

Les différents résultats obtenus sont rassemblés dans le tableau 2.

Ces vernis sont tous stables à 50° C pendant au moins un mois. A température ambiante on ne constate pas de sédimentation ni de séparation de phases, sur une longue période.

Les compositions de vernis selon l'invention présentent de bonnes propriétés sur l'ongle. En particulier, ils peuvent être conservés trois jours sur l'ongle sans usure prématurée, ni écaillage, ni décollement dans l'eau. Ces vernis se démaquillent ensuite facilement avec des dissolvants classiques à base d'esters ou de cétones.

En ce qui concerne la base aqueuse selon l'exemple 2, elle s'applique en une couche unique sur l'ongle et peut être recouverte par une ou deux couches de vernis classique avec solvants. Elle permet ainsi de protéger l'ongle des effets néfastes de certains composants des vernis à base de solvants, l'aspect final et la tenue sur l'ongle d'un tel système étant supérieurs aux systèmes actuels.

Les viscosités et la stabilité sont pratiquement équivalentes d'une formule à l'autre.

Le séchage dépend de la vitesse d'évaporation de l'eau et des coalescents. La rétention du coalescent semble moindre avec la suspension réticulée.

Le résultat le plus important est la vitesse de durcissement qui est plus rapide en présence d'émulsion réticulée. De plus, on a remarqué que la dureté ne continue pas à augmenter avec le temps. Le film reste tenace et souple sur l'ongle sans écaillage. Il est ainsi possible d'obtenir, rapidement sur l'ongle, un film résistant à l'usure et à l'abrasion, sans craindre d'avoir un film, après 24 heures, très dur et cassant, avec écaillage et perte d'adhérence.

Cette consistance du film sec permet d'avoir une bonne adhérence sur l'ongle. Le film n'est pas mou les premières heures avec usures et décollement, ni dur avec écaillage après 24 heures. On obtient rapidement un film dur et élastique, qui peut être gardé plusieurs jours sur l'ongle.

Les résultats d'adhérence et de tenue sur l'ongle sont directement liés à ces propriétés de dureté/souplesse recherchées.

Les résultats de l'exemple comparatif 6 ne sont pas bons car l'émulsion n'est pas suffisamment dure pour obtenir la résistance recherchée. De plus, on obtient un film qui sèche très rapidement mais qui se ramollit sur l'ongle au cours du temps. La présence de la suspension (A) est donc essentielle.

### Exemple comparatif 7

On a préparé une formulation conforme à l'exemple 1, en omettant la suspension (B). Le vernis obtenu a une durée de durcissement longue due à l'absence du polymère préréticulé et il est difficile à enlever.

Les essais susmentionnés montrent donc que la présence simultanée des suspensions (A) et (B) est essentielle pour obtenir les avantages indiqués.

**Tableau 1**

| **Exemple** | | **1** | **2** | **3** | **4** | **5 (comp.)** | **6 (comp.)** |
|---|---|---|---|---|---|---|---|
| Suspension (A) | Joncryl 538 (à 46 % en solide) | 38 | 35 | 34 | 36 | 34 | |
| Coalescent | Dipropylène glycol diméthyl ether | 6 | 5 | | | | |
| Coalescent | Dipropylène glycol monométhyléther | | | 3 | / | 3 | 3 |
| Plastifiant | Monoisobutyrate de triméthyl pentanediol | 4 | 3 | 4 | 5 | 4 | |
| Actif | Acétate de Vitamine E | | 0,1 | | | | |
| Suspension (B) | Joncryl 8224 (à 45 % en solide) | 35 | 45 | 45 | 44 | | 80 |
| Suspension | Non réticulée à Tg = 46° C et TMF = 45° C | | | | | 45 | |
| Epaississant | Silicate de Mg et Na | 0,6 | | 0,6 | 0,6 | 0,6 | 0,6 |
| Conservateur | Solution de Parabens dans le phénoxyéthanol | 0,5 | 0,5 | 0,5 | 0,5 | 0,5 | 0,5 |
| Epaississant | Epaississant organique | 0,5 | 0,8 | 0,8 | 0,8 | 0,8 | 0,8 |
| Additif | Agent siliconé de tension et glissance | 0,5 | | 0,5 | 0,5 | 0,5 | 0,5 |
| Solution (C) | Joncryl HPD 71 E (à 25 % en solide) | 3 | | 3 | 3 | 3 | 3 |
| Pigment | DC red 34 | 0,3 | | 0,3 | 0,3 | 0,3 | 0,3 |
| Pigment | DC red 7 | 0,3 | | 0,3 | 0,3 | 0,3 | 0,3 |
| Pigment | Mica titane | 2 | | 2 | 2 | 2 | 2 |
| Pigment | DC Red 30 | | 0,01 | | | | |
| Diluant | Eau déminéralisée, complément à 100 | QS | QS | QS | QS | QS | QS |

**Tableau 2**

| **Exemple** | | **1** | **2** | **3** | **4** | **5 (comp.)** | **6 (comp.)** |
|---|---|---|---|---|---|---|---|
| Viscosité Brookfield | Vitesse 6 tours | 1620 | 860 | 1700 | 1200 | 1540 | 1485 |
| (mPa.s) | Vitesse 60 tours | 620 | 740 | 895 | 672 | 732 | 760 |
| Séchage (min) | A 35° C | 5 min 30 | 5 min 45 | 7 min 15 | 6 min 15 | 8 min | 5 min 15 |
| Dureté Persoz | Après 3 h de séchage | 150 | 145 | 160 | 165 | 125 | 155 |
| (secondes) | Après 24 heures de séchage | 210 | 190 | 198 | 191 | 175 | 175 |
| Adhérence | (0 excellente, 5 mauvaise) | 0/1 | 0/1 | 0/1 | 0/1 | 1 | 1 |
| Brillant | | 79 | 82 | 88 | 80 | 82 | 78 |
| Stabilité à 50° C | | ok | ok | ok | ok | ok | ok |
| Tenue sur l'ongle | A 3 jours | ok | ok | ok | ok | usure | Usure |
| aspect | | brillant | avec vernis solvant | brillant | brillant | mat | ramollissement |

## Revendications

1. Vernis à ongles sous forme d'une composition aqueuse, comprenant un mélange d'au moins
- une suspension aqueuse (A) de nanoparticules dures, de taille de 40 à 80 nm, d'un polymère acrylique à température de transition vitreuse supérieure ou égale à 55° C, en combinaison avec un plastifiant dudit polymère choisi parmi l'adipate de dibutyle, le sébacate de dibutyle, l'acétyl citrate de tributyle, le citrate de triéthyle, ou un butyrate d'alkyldiol, ou un mélange de ceux-ci ;
- et d'au moins une suspension aqueuse (B) de nanoparticules, de taille de 50 à 80 nm de polymère acrylique préréticulé, renfermant des monomères di- ou trifonctionnels et présentant une température de formation de film inférieure à 15° C.

2. Vernis à ongles selon la revendication 1, **caractérisé en ce qu'**il renferme également une solution (C) d'un polymère acrylique de faible poids moléculaire inférieur à 20 000 environ, à indice d'acide supérieur à 200 milligrammes de KOH par gramme de polymère, neutralisée jusqu'à un pH entre 7 et 8,5 environ.

3. Vernis à ongles selon l'une quelconque des revendications 1 ou 2, **caractérisé en ce que** les nanoparticules de la suspension (A) sont à base d'un polymère de poids moléculaire supérieur à 200 000 environ.

4. Vernis à ongles selon l'une quelconque des revendications précédentes, **caractérisé en ce que** la suspension aqueuse (A) est combinée à un solvant coalescent du polymère de la suspension (A) qui est un éther de glycol choisi parmi le propylène glycol n-butyl éther, le dipropylène glycol méthyl ether, le dipropylène glycol diméthyl éther, le tri propylène glycol méthyl éther, ou un mélange de ceux-ci.

5. Vernis à ongles selon l'une des revendications précédentes, **caractérisée en ce que** le solvant plastifiant du polymère de la suspension (A) est le mono isobutyrate de triméthyl pentanediol.

6. Vernis à ongles selon l'une quelconque des revendications précédentes, **caractérisé en ce que** les solvants coalescent et/ou plastifiant du polymère de la suspension (A) sont utilisés chacun dans des proportions jusqu'à 15 %, de préférence comprises entre 5 et 15 % en poids du polymère.

7. Vernis à ongles selon l'une quelconque des revendications précédentes, **caractérisé en ce que** les nanoparticules de la suspension (B) présentent un taux de monomères di- ou trifonctionnels compris entre 0,5 et 5%.

8. Vernis à ongles selon l'une quelconque des revendications précédentes, **caractérisé en ce que** les monomères et polymères acryliques sont à base de styrène, de méthyl styrène, d'acrylate de méthyle, de méthacrylate de méthyle, d'acrylate de butyle, de méthacrylate de butyle, de méthacrylate d'hydroxyéthyle, de méthacrylate, d'hydroxypropyle, ou un mélange de ceux-ci.

9. Vernis à ongles selon l'une quelconque des revendications précédentes, **caractérisé en ce qu'**il renferme entre 12 et 24 % en poids de nanoparticules de la suspension (A), entre 8 et 16 % en poids de nanoparticules de la suspension (B), et entre 0,2 et 1 % en poids de polymère sec de l'éventuelle solution (C).

10. Vernis à ongles selon l'une quelconque des revendications 2 à 9, **caractérisé en ce qu'**il renferme des colorants solubles dans l'eau et/ou des pigments dispersés dans la solution aqueuse acrylique (C).

11. Vernis à ongles selon l'une quelconque des revendications précédentes, **caractérisé en ce qu'**il renferme des agents épaississants minéraux ou organiques, des agents antimousses siliconés ou non, des agents conservateurs, des agents d'étalement et/ou de glissance, des parfums, des neutralisants, ou des additifs actifs pour les soins des ongles.

12. Vernis à ongles selon l'une quelconque des revendications précédentes, ayant la composition suivante :
| | | |
|---|---|---|
| Suspension (A) | Joncryl 538 (à 46% en solide) Suspension aqueuse de polymères acides et esters(méth)acryliques/styrène présentant une température de formation de film (TMF) de 60°C et une température de transition vitreuse (Tg) de 64 °C | 38 |
| Coalescent | Dipropylène glycol diméthyl éther | 6 |
| Plastifiant | Monoisobutyrate de triméthyl pentanediol | 4 |
| Suspension (B) | Joncryl 8224 (à 45% en solide) Suspension acrylique présentant une TMF de 10°C et une Tg voisine de 46 °C. | 35 |
| Epaississant minéral | Silicate de magnésium et de sodium | 0,6 |
| Conservateur | Solution de Parabens dans le phénoxyéthanol | 0,5 |
| | Epaississant organique | 0,5 |
| | Agent siliconé de tension et glissance | 0,5 |
| Solution (C) | Joncryl HPD 71 E (à 25% en solide) Solution aqueuse d'un sel d'ammonium de polymères styrènes/acides et esters (méth)acryliques. | 3 |
| Pigments | DC red 34 | 0,3 |
| | DC red 7 | 0,3 |
| | Mica titane | 2 |
| Eau | Eau déminéralisée complément à 100 | |

13. Vernis à ongles selon l'une quelconque des revendications 1 à 10, ayant la composition suivante :
| | | |
|---|---|---|
| Suspension (A) | Joncryl 538 (à 46% en solide) Suspension aqueuse de polymères acides et esters(méth)acryliques/styrène présentant une température de formation de film (TMF) de 60°C et une température de transition vitreuse (Tg) de 64 °C | 35 |
| Coalescent | Dipropylène glycol diméthyl éther | 5 |
| Plastifiant | Monoisobutyrate de triméthyl pentanediol | 3 |
| | Acétate de vitamine E | 0,1 |
| Suspension (B) | Joncryl 8224 (à 45% en solide) Suspension acrylique présentant une TMF de 10°C et une Tg voisine de 46 °C. | 45 |
| Conservateur | Solution de Parabens dans le phénoxyéthanol | 0,5 |
| | Epaississant organique | 0,8 |
| Solution (C) | Joncryl HPD 71 E (à 25% en solide) Solution aqueuse d'un sel d'ammonium de polymères styrènes/acides et esters (méth)acryliques. | 2 |
| Pigments | DC red 30 | 0,01 |
| | Eau déminéralisée complément à 100 | |

14. Vernis à ongles selon l'une quelconque des revendications précédentes, ayant la composition suivante :
| | | |
|---|---|---|
| Suspension (A) | Joncryl 538 (à 46% en solide) Suspension aqueuse de polymères acides et esters(méth)acryliques/styrène présentant une température de formation de film (TMF) de 60°C et une température de transition vitreuse (Tg) de 64 °C | 34 |
| Coalescent | Dipropylène glycol mono méthyl éther | 3 |
| Plastifiant | Monoisobutyrate de triméthyl pentanediol | 4 |
| Suspension (B) | Joncryl 8224 (à 45% en solide) Suspension acrylique présentant une TMF de 10°C et une Tg voisine de 46 °C. | 45 |
| Epaississant minéral | Silicate de magnésium et de sodium | 0,6 |
| Conservateur | Solution de Parabens dans le phénoxyéthanol | 0,5 |
| | Epaississant organique | 0,8 |
| | Agent siliconé de tension et glissance | 0,5 |
| Solution (C) | Joncryl HPD 71 E (à 25% en solide) Solution aqueuse d'un sel d'ammonium de polymères styrènes/acides et esters (méth)acryliques. | 3 |
| Pigments | DC red 34 | 0,3 |
| | DC red 7 | 0,3 |
| | Mica titane | 2 |
| Eau | Eau déminéralisée complément à 100 | |

15. Vernis à ongles selon l'une quelconque des revendications 1 à 10, ayant la composition suivante :
| | | |
|---|---|---|
| Suspension (A) | Joncryl 538 (à 46% en solide) Suspension aqueuse de polymères acides et esters(méth)acryliques/styrène présentant une température de formation de film (TMF) de 60°C et une température de transition vitreuse (Tg) de 64 °C | 36 |
| Plastifiant | Monoisobutyrate de triméthyl pentanediol | 5 |
| Suspension (B) | Joncryl 8224 (à 45% en solide) Suspension acrylique présentant une TMF de 10°C et une Tg voisine de 46 °C. | 44 |
| Epaississant minéral | Silicate de magnésium et de sodium | 0,6 |
| | Agent siliconé de tension et glissance | 0,5 |
| Conservateur | Solution de Parabens dans le phénoxyéthanol | 0,5 |
| | Epaississant organique | 0,8 |
| Solution (C) | Joncryl HPD 71 E (à 25% en solide) Solution aqueuse d'un sel d'ammonium de polymères styrènes/acides et esters (méth)acryliques. | 3 |
| Pigments | DC red 34 | 0,3 |
| | DC red 7 | 0,3 |
| | Mica titane | 2 |
| Eau | Eau déminéralisée complément à 100 | |

## Claims

1. A nail varnish in the form of an aqueous composition, comprising a mixture of at least
- one aqueous suspension (A) of hard nanoparticles, with a size from 40 to 80 nm, of an acrylic polymer with a glass transition temperature greater than or equal to 55°C, in combination with a plasticizer of said polymer selected from dibutyl adipate, dibutyl sebacate, tributyl acetyl citrate, triethyl citrate or an alkyldiol butyrate, or a mixture thereof;
- and of at least one aqueous suspension (B) of nanoparticles, with a size from 50 to 80 nm, of a pre-crosslinked acrylic polymer containing di- or tri-functional monomers and having a film formation temperature below 15°C.

2. The nail varnish according to claim 1, **characterized in that** it also contains a solution (C) of an acrylic polymer with low molecular weight of less than about 20,000, with an acid index of more than 200 milligrams of KOH per gram of polymer, neutralized up to a pH between 7 and about 8.5.

3. The nail varnish according to any of claims 1 or 2, **characterized in that** the nanoparticles of the suspension (A) are based on a polymer with a molecular weight greater than about 200,000.

4. The nail varnish according to any of the preceding claims, **characterized in that** the aqueous suspension (A) is combined with a coalescent solvent of the polymer of the suspension (A) which is a glycolether selected from propyleneglycol n-butyl ether, dipropylene glycol methyl ether, dipropylene glycol dimethyl ether, tripropylene glycol methyl ether or a mixture thereof.

5. The nail varnish according to any of the preceding claims, **characterized in that** the plasticizing solvent of the polymer of the suspension (A) is trimethyl pentane-diol monoisobutyrate.

6. The nail varnish according to any of the preceding claims, **characterized in that** coalescent and/or plasticizing solvents of the polymer of the suspension (A) are each used in proportions up to 15%, preferably comprised between 5 and 15% by weight of the polymer.

7. The nail varnish according to any of the preceding claims, **characterized in that** the nanoparticles of the suspension (B) have a level of di- or tri-functional monomers comprised between 0.5 and 5%.

8. The nail varnish according to any of the preceding claims, **characterized in that** the acrylic monomers and polymers are based on styrene, methyl styrene, methyl acrylate, methyl methacrylate, butyl acrylate, butyl methacrylate, hydroxyethyl methacrylate, hydroxypropyl methacrylate or a mixture thereof.

9. The nail varnish according to any of the preceding claims, **characterized in that** it contains between 12 and 24% by weight of nanoparticles of the suspension (A), between 8 and 16% by weight of nanoparticles of the suspension (B) and between 0.2 and 1% by weight of dry polymer of the optional solution (C).

10. The nail varnish according to any of claims 2 to 9, **characterized in that** it contains water-soluble dyes and/or pigments dispersed in the acrylic aqueous solution (C).

11. The nail varnish according to any of the preceding claims, **characterized in that** it contains mineral or organic thickeners, antifoam agents either with silicone or not, preservatives, spreading and/or smoothing agents, perfumes, neutralizing agents, or active additives for nail care.

12. The nail varnish according to any of the preceding claims, **characterized in that** having the following composition:
| | | |
|---|---|---|
| Suspension (A) | Joncryl 538 (46% solids) Aqueous suspension of ((meth)acrylic acid and ester) / styrene polymers having a minimum film-forming temperature (MFT) of 60°C and a glass transition temperature (Tg) of 64°C | 38 |
| Coalescent | Dipropylene glycol dimethylether | 6 |
| Plasticizer | Trimethyl pentanediol monoisobutyrate | 4 |
| Suspension (B) | Joncryl 8224 (45% solids) Acrylic suspension having a MFT of 10°C and a Tg around 46°C. | 35 |
| Mineral thickener | Magnesium and sodium silicate | 0.6 |
| Preservative | Solution of parabens in phenoxyethanol | 0.5 |
| | Organic thickener | 0.5 |
| | Silicone surfactant and slip agent | 0.5 |
| Solution (C) | Joncryl HPD 71E (25% solids) Aqueous solution of an ammonium salt of styrene/ ((meth)acrylic acid and ester) polymers. | 3 |
| Pigments | DC red 34 | 0.3 |
| | DC red 7 | 0.3 |
| | Titanium mica | 2 |
| Water | Demineralized water: balance to 100 | |

13. The nail varnish according to any of claims 1 to 10 having the following composition:
| | | |
|---|---|---|
| Suspension (A) | Joncryl 538 (46% solids) | 35 |
| | Aqueous suspension of ((meth)acrylic acid and ester) / styrene polymers having a minimum film-forming temperature (MFT) of 60°C and a glass transition temperature (Tg) of 64°C | |
| Coalescent | Dipropylene glycol dimethyl ether | 5 |
| Plasticizer | Trimethyl pentanediol monoisobutyrate | 3 |
| | Vitamin E acetate | 0.1 |
| Suspension (B) | Joncryl 8224 (45% solids) | 45 |
| | Acrylic suspension having a MFT of 10°C and a Tg around 46°C. | |
| Preservative | Solution of parabens in phenoxyethanol | 0.5 |
| | Organic thickener | 0.8 |
| Solution (C) | Joncryl HPD 71E (25% solids) Aqueous solution of an ammonium salt of styrene/ ((meth)acrylic acid and ester) polymers. | 2 |
| Pigments | DC red 30 | 0.01 |
| | Demineralized water: balance to 100 | |

14. The nail varnish according to any of the preceding claims having the following composition:
| | | |
|---|---|---|
| Suspension (A) | Joncryl 538 (46% solids) | 34 |
| | Aqueous suspension of ((meth)acrylic acid and ester) / styrene polymers having a minimum film-forming temperature (MFT) of 60°C and a glass transition | |
| | temperature (Tg) of 64°C | |
| Coalescent | Dipropylene glycol dimethyl ether | 3 |
| Plasticizer | Trimethyl pentanediol monoisobutyrate | 4 |
| Suspension (B) | Joncryl 8224 (45% solids) | 45 |
| | Acrylic suspension having a MFT of 10°C and a Tg around 46°C. | |
| Mineral thickener | Magnesium and sodium silicate | 0.6 |
| Preservative | Solution of parabens in phenoxyethanol | 0.5 |
| | Organic thickener | 0.8 |
| | Silicone surfactant and slip agent | 0.5 |
| Solution (C) | Joncryl HPD 71E (25% solids) | 3 |
| | Aqueous solution of an ammonium salt of styrene/ ((meth)acrylic acid and ester) polymers. | |
| Pigments | DC red 34 | 0.3 |
| | DC red 7 | 0.3 |
| | Titanium mica | 2 |
| Water | Demineralized water: balance to 100 | |

15. The nail varnish according to any of claims 1 to 10, having the following composition:
| | | |
|---|---|---|
| Suspension (A) | Joncryl 538 (46% solids) | 36 |
| | Aqueous suspension of ((meth)acrylic acid and ester) / styrene polymers having a minimum film-forming temperature (MFT) of 60°C and a glass transition temperature (Tg) of 64°C | |
| Plasticizer | Trimethyl pentanediol monoisobutyrate | 5 |
| Suspension (B) | Joncryl 8224 (45% solids) | 44 |
| | Acrylic suspension having a MFT of 10°C and a Tg around 46°C. | |
| Mineral thickener | Magnesium and sodium silicate | 0.6 |
| | Silicone surfactant and slip agent | 0.5 |
| Preservative | Solution of parabens in phenoxyethanol | 0.5 |
| | Organic thickener | 0.8 |
| Solution (C) | Joncryl HPD 71E (25% solids) | 3 |
| | Aqueous solution of an ammonium salt of styrene/ ((meth)acrylic acid and ester) polymers. | |
| Pigments | DC red 34 | 0.3 |
| | DC red 7 | 0.3 |
| | Titanium mica | 2 |
| Water | Demineralized water: balance to 100 | |

## Patentansprüche

1. Nagellack in Form einer wässrigen Zusammensetzung, umfassend eine Mischung aus mindestens
- eine wässrige Suspension (A) von gehärteten Nanopartikeln der Größe 40 bis 80 nm mit einem Acrylpolymer mit einer Glaswandlungstemperatur über oder gleich 55 °C in Kombination mit einem Weichmacher des Polymers, ausgewählt unter dem Dibutyladipat, dem Dibutylsebakat, dem Tributylacetylcitrat, dem Triethylcitrat oder einem Alkyldiol-butyrat oder einer Mischung dieser;
und mindestens eine wässrige Suspension (B) von Nanopartikeln mit der Größe 50 bis 80 nm aus vorvernetztem Acrylpolymer, das di- oder trifunktionale Monomere einschließt und eine Filmbildungstemperatur von weniger als 15 °C aufweist.

2. Nagellack nach Anspruch 1, **dadurch gekennzeichnet, dass** er auch eine Lösung (C) eines Acrylpolymers mit geringem Molekulargewicht unter ungefähr 20 000, mit einer Säurezahl von mehr als 200 Milligramm KOH pro Gramm Polymer, neutralisiert bis zu einem pH zwischen ungefähr 7 und 8,5, aufweist.

3. Nagellack nach einem der Ansprüche 1 oder 2, **dadurch gekennzeichnet, dass** die Nanopartikel der Suspension (A) auf einem Polymer mit einem Molekulargewicht von mehr als ungefähr 200 000 basieren.

4. Nagellack nach einem der vorhergehenden Ansprüche, **dadurch gekennzeichnet, dass** die wässrige Suspension (A) mit einem Koaleszenzlösemittel des Polymers der Suspension (A) kombiniert wird, wobei es sich um ein Glykolether handelt, ausgewählt aus Propylenglykol-n-butylether, Dipropylenglykolmethylether, Dipropylenglykoldimethylether, Tripropylenglykolmethylether, oder einer Mischung dieser.

5. Nagellack nach einem der vorhergehenden Ansprüche, **dadurch gekennzeichnet, dass** das Weichmacher-Lösemittel des Polymers der Suspension (A) Trimethylpentandiol-monoisobutyrat ist.

6. Nagellack nach einem der vorhergehenden Ansprüche, **dadurch gekennzeichnet, dass** die Koaleszenzlösemittel und/oder die Weichmacher-Lösemittel des Polymers der Suspension (A) jeweils in Proportionen bis 15, vorzugsweise zwischen 5 und 15 Gew% des Polymers verwendet werden.

7. Nagellack nach einem der vorhergehenden Ansprüche, **dadurch gekennzeichnet, dass** die Nanopartikel der Suspension (B) einen Anteil an di- oder trifunktionalen Monomeren zwischen 0,5 und 5 % aufweisen.

8. Nagellack nach einem der vorhergehenden Ansprüche, **dadurch gekennzeichnet, dass** die Acrylmonomere und -polymere auf Styren, Methylstyren, Methylacrylat, Methylmethacrylat, Butylacrylat, Butylmethacrylat, Hydroxyethylmethacrylat, Hydroxpropylmethacrylat; oder einer Mischung dieser basieren.

9. Nagellack nach einem der vorhergehenden Ansprüche, **dadurch gekennzeichnet, dass** er zwischen 12 und 24 Gew% Nanopratikel der Suspension (A), zwischen 8 und 16 Gew% Nanopartikel der Suspension (B) und zwischen 0,2 und 1 Gew% Trockenpolymer der eventuellen Lösung (C) einschließt.

10. Nagellack nach einem der Ansprüche 2 bis 9, **dadurch gekennzeichnet, dass** er wasserlösliche Farbstoffe und/oder in der wässrigen Acryllösung (C) dispergierte Pigmente einschließt.

11. Nagellack nach einem der vorhergehenden Ansprüche, **dadurch gekennzeichnet, dass** er mineralische oder organische Verdickungsmittel, silikonierte oder nicht silikonierte Schaumverhütungsmittel, Konservierungsmittel, Spreitungs- und/oder Glättungsmittel, Duftstoffe, Neutralisierungsmittel oder aktive Zusatzstoffe für die Pflege der Nägel einschließt.

12. Nagellack nach einem der vorhergehenden Ansprüche mit der folgenden Zusammensetzung:
| | | |
|---|---|---|
| Suspension (A) | Joncryl 538 (mit 46 % Festgehalt) | 38 |
| | Wässrige Suspension von sauren Polymeren und (Meth)acrylestern/Styren, die eine Filmbildungstemperatur (TMF) von 60 °C und eine Glaswandlungstemperatur (Tg) von 64 °C aufweisen. | |
| Koaleszenz | Dipropylenglykoldimethylether | 6 |
| Weichmacher | Trimethylpentandiol-monoisobutyrat | 4 |
| Suspension (B) | Joncryl 8224 (mit 45 % Festgehalt) Acrylische Suspension, die eine TMF von 10 °C und eine Tg von 46 °C aufweist. | 35 |
| Mineralisches Verdickungsmittel | Magnesium- und Natriumsilikat | 0,6 |
| Konservierungsmittel | Lösung von Parabenen in Phenoxyethanol | 0,5 |
| | Organisches Verdickungsmittel | 0,5 |
| | Silikoniertes Spannungs- und Gleitmittel | 0,5 |
| Lösung (C) | Joncryl HPD 71 (mit 25 % Festgehalt) | 3 |
| | Wässrige Lösung eines Ammoniumsalzes von Styren-/Säure**polymeren und** (Meth)acrylestern. | |
| Pigmente | DC red 34 | 0,3 |
| | DC red 7 | 0,3 |
| | Mica-Titan | 2 |
| Wasser | Entmineralisierter Wasserzusatz bei 100 | |

13. Nagellack nach einem der Ansprüche 1 bis 10 mit der folgenden Zusammensetzung:
| | | |
|---|---|---|
| Suspension (A) | Joncryl 538 (mit 46 % Festgehalt) | 35 |
| | Wässrige Suspension von sauren Polymeren und (Meth)acrylestern/Styren, **die eine** Filmbildungstemperatur (TMF) von 60 °C und eine Glaswandlungstemperatur (Tg) von 64 °C aufweisen. | |
| Koaleszenz | Dipropylenglykoldimethylether | 5 |
| Weichmacher | Trimethylpentandiol-monoisobutyrat | 3 |
| | Vitamin-E-Acetat | 0,1 |
| Suspension (B) | Joncryl 8224 (mit 45 % Festgehalt) | 45 |
| | Acrylische Suspension, die eine TMF von 10 °C und eine Tg von ungefähr 46 °C aufweist. | |
| Konservierungsmittel | Lösung von Parabenen in Phenoxyethanol | 0,5 |
| | Organisches Verdickungsmittel | 0,8 |
| Lösung (C) | Joncryl HPD 71 E (mit 25 % Festgehalt) | 2 |
| | Wässrige Lösung eines Ammoniumsalzes von Styren-/Säurepolymeren und (Meth)acrylestern. | |
| Pigmente | DC red 30 | 0,01 |
| | Entmineralisierter Wasserzusatz bei 100 | |

14. Nagellack nach einem der vorhergehenden Ansprüche mit der folgenden Zusammensetzung:
| | | |
|---|---|---|
| | Joncryl 538 (mit 46 % Festgehalt) | 34 |
| Suspension (A) | Wässrige Suspension von sauren Polymeren und (Meth)acryles**tern/Styren, die eine** Filmbildungstemperatur (TMF) von 60 °C und eine Glaswandlungstemperatur (Tg) von 64 °C aufweisen. | |
| Koaleszenz | Dipropylenglykolmonomethylether | 3 |
| Weichmacher | Trimethylpentandiol-monoisobutyrat | 4 |
| Suspension (B) | Joncryl 8224 (mit 45 % Festgehalt) | 45 |
| | Acrylische Suspension, die eine TMF von 10 °C und eine Tg von ungefähr 46 °C aufweist. | |
| Mineralisches Verdickungsmittel | Magnesium- und Natriumsilikat | 0,6 |
| Konservierungsmittel | Lösung von Parabenen in Phenoxyethanol | 0,5 |
| | Organisches Verdickungsmittel | 0,8 |
| | Silikoniertes Spannungs- und Gleitmittel | 0,5 |
| Lösung (C) | Joncryl HPD 71 E (mit 25 % Festgehalt) | 3 |
| | Wässrige Lösung eines Ammoniumsalzes von Styren-/ Säurepolymeren und (Meth)acrylestern. | |
| Pigmente | DC red 34 | 0,3 |
| | DC red 7 | 0,3 |
| | Mica-Titan | 2 |
| Wasser | Entmineralisierter Wasserzusatz bei 100 | |

15. Nagellack nach einem der Ansprüche 1 bis 10 mit der folgenden Zusammensetzung:
| | | |
|---|---|---|
| Suspension (A) | Joncryl 538 (mit 46 % Festgehalt) | 36 |
| | Wässrige Suspension von sauren Polymeren und (Meth)acrylies**tern/Styren, die eine** Filmbildungstemperatur (TMF) von 60 °C und eine Glaswandlungstemperatur (Tg) von 64 °C aufweisen. | |
| Weichmacher | Trimethylpentandiol-monoisobutyrat | 5 |
| Suspension (B) | Joncryl 8224 (mit 45 % Festgehalt) | 44 |
| | Acrylische Suspension, die eine TMF von 10 °C und eine Tg von ungefähr 46 °C aufweist. | |
| Mineralisches Verdickungsmittel | Magnesium- und Natriumsilikat | 0,6 |
| | Silikoniertes Spannungs- und Gleitmittel | 0,5 |
| Konservierungsmittel | Lösung von Parabenen in Phenoxyethanol | 0,5 |
| | Organisches Verdickungsmittel | 0,8 |
| Lösung (C) | Joncryl HPD 71 E (mit 25 % Festgehalt) | 3 |
| | Wässrige Lösung eines Ammoniumsalzes von Styren-/ Säurepolymeren und (Meth)acrylestern. | |
| Pigmente | DC red 34 | 0,3 |
| | DC red 7 | 0,3 |
| | Mica-Titan | 2 |
| Wasser | Entmineralisierter Wasserzusatz bei 100 | |
